# EUROPEAN PATENT SPECIFICATION

(11) **EP 1 808 138 B1**
(45) Date of publication and mention of the grant of the patent: **10.09.2014**
(21) Application number: 06008538.8
(22) Date of filing: 25.04.2006
(51) Int. Cl.: A61B 17/17, A61B 17/88, A61B 17/00, A61B 17/80

(54) **Medical system and system parts for spatially adjusting an aiming device relative to a body implant**
Medizinisches System und dazugehörige Systemteile zum räumlichen Einstellen eines Zielgerätes an einem Implantat
Système médical pour ajuster un guide de positionnement par rapport à un implant et éléments de ce système

(30) Priority: 17.01.2006 US 759376 P
(43) Date of publication of application: 18.07.2007
(73) Proprietor: Stryker Trauma GmbH, 24232 Schönkirchen/Kiel (DE)
(72) Inventor: Prien, Ole, 24145 Kiel (DE)
(74) Representative: Dilg, Andreas

(56) References cited:
- WO-A-2005/089660
- DE-A1- 10 131 992
- US-A- 5 766 174
- US-A1- 2004 034 356
- US-A1- 2005 085 818
- US-B2- 6 989 012

## Description

### FIELD OF INVENTION

The present invention relates to the field of surgery instruments. In particular, the present invention relates to a medical system for detachably establishing a spatial orientation between a body implant and an aiming device. More particular, the present invention relates to a medical system and parts of the system, which allow for a precise establishing of a relative spatial orientation between a bone stabilization implant and an aiming device for anchoring the implant to an internal part of a body.

The invention further relates to a method for detachably establishing a spatial orientation between a body implant and an aiming device, in particular between a bone stabilization implant and an aiming device for anchoring the implant to an internal part of a body.

### BACKGROUND OF THE INVENTION

In order to allow for a reliable stabilization of a broken bone in its normal position, special bone stabilizing implants are frequently used. Such implants are for instance metal plates, which are made e.g. from surgery steel. Plates used for such purposes are usually fixed to the bone parts by means of threaded screws, which are driven into the bone tissue after so-called pre-drilled or pilot-drilled holes have been generated within the bone tissue. These pre-drilled holes allow for a reliable screwing procedure whereby the risk of further destroying the bone with the screw is significantly reduced.

In order to facilitate the drilling of these pre-drilled holes there are known so-called aiming devices or aiming blocks, which work like a drilling jig. Thereby, an aiming device is detachably fixed to the metal plate in a spatial precise position.

Aiming devices are also used in connection with locking nails, which are driven into intra medullary tissue. Thereby, the position of a cross bore within the interlocking nail can be determined precisely. The cross bore is adapted to accommodate a fixing screw, which is driven crosswise through the corresponding bone section.

US 5,766,174 (The preamble of claim 1 is based on this document) discloses an intramedulary rod having a cylindrical proximal portion and a rectangular distal portion with a tapered blade-like portion. The intramedulary rod provides rotational stability and resistance to axial migration. Proximal and distal trans-fixation holes may be provided in the intramedulary rod. The rod comprises a proximal end with a shallow threaded portion that includes a pair of opposed notches for an engagement with an anvil which is provided with correspondingly opposed tabs.

DE 101 31 992 discloses a bone plate including two notches at opposite side surfaces for engagement with an adapter device which in turn can be connected to an aiming device by means of a pin-like structure at its proximal end engaging with a corresponding recess in the aiming device. At the distal end, the adapter device comprises two opposed protrusions corresponding to the recesses in the side surfaces of the bone plate.

US 6,224,601 B discloses the use of an aiming device in an osteosynthesis auxiliary for the treatment of subtrochanteric, peritochanteric and femoral-neck fractures.

There may be a need for a medical system, medical system parts and a method for spatially adjusting an aiming device relative to a body implant, which allow for an easy, for a quick and for a precise relative positioning of an aiming device with respect to a body implant.

### SUMMARY OF THE INVENTION

In order to fulfill the need defined above there are provided a body implant, an adaptor device, a medical system and a method for detachably establishing a spatial orientation between a body implant and an aiming device as set forth in the independent claims.

According to an aspect of the invention there is provided a body implant, in particular a bone stabilizing implant. The body implant comprises (a) through holes for anchoring the implant to an internal part of a body by means of fastening elements and (b) a contour portion, which is designed in such a manner that an adaptor device for establishing a spatial orientation between the body implant and an aiming device can be detachably fastened to the body implant by means of a mechanic form-fit connection. The contour portion comprises a central opening and three grooves. Thereby, the central opening might interact with the adaptor device like a mortise and a tenon such that the relative position between the implant and the adaptor device is defined precisely. The central opening preferably has a circular respectively a cylindrical shape such that during manufacturing of the body implant the opening can easily be formed within the implant by means of a simple drilling procedure.

The groove may be formed in such a manner that it might mutually interact with a corresponding protrusion from the adaptor device. This interaction may be similar to the well-known mechanical interaction between tongue and groove. Thereby, also a rotational movement of the adaptor device with respect to the body implant is effectively prevented.

This aspect of the present invention is based on the idea that a form-fit connection between the body implant and the adaptor device can be realized if the adaptor device and the corresponding contour portion formed at the body implant spatially interact with each other in such a manner that these parts mutually intervene and/or engage with each other. Thereby, a precise relative spatial orientation of the body implant and the adaptor device can be maintained.

The body implant may be a plate for stabilizing a broken bone in its normal position. The plate may be used for stabilizing the proximal tibia. Typically, the fastening elements are screws, which can be driven into different portions of a broken bone.

The aiming device has a structure, which is related to at least some of the through holes formed within the body implant. Such a structure typically represents a drilling jig in order to allow for an easy and for a precise pre-drilling of holes within the broken bone. This pre-drilling is carried out through the through holes formed in the body implant such that it is guaranteed that the pre-drilled holes spatially correspond to the through holes of the body implant.

During an operation of a patient it has also to be ensured that also the adaptor device and the aiming device are rigidly fixed to each other. Therefore, the form locked adaptor device provides a mechanically stable platform for the aiming device.

According to the first aspect of the present invention the central opening is a through hole.

This provides the advantage that the central opening may be formed by means of an in particular simple drilling procedure, because it is not necessary to take care of the actual drilling depth during the drilling procedure.

According to the first aspect of the present invention the contour portion comprises two further grooves. This may provide the advantage that the relative spatial position between the adaptor device and the body implant can be established even more precisely and in a more reliably way.

In this respect it is clear that the adaptor device has to be formed in a complementary shape with respect to the contour portion. Thereby, a form-fit is possible not only with respect to the central opening and to one groove but with respect to the central opening and to the three grooves.

According to the first aspect of the present invention the grooves are arranged in an asymmetric manner with respect to the central opening. This may provide the advantage that the adaptor device and the body implant can be mechanically connected to each other only in a predefined relative orientation. Therefore, an accidentally wrong assembling of these parts can be prevented effectively.

According to an embodiment of the present invention the contour portion comprises a locking element which is designed in such a manner that the adaptor device is lockable to the body implant by means of a locking mechanism assigned to the adaptor device.

The provision of a locking element has the advantage that an unintentional opening of the mechanical form-fit connection between the adaptor device and the body implant may be prevented effectively. Of course, the adaptor device may be removed from the implant by an appropriate procedure after a surgery procedure has been successfully carried out. Thereby, the surgery procedure might have been taken benefit from using the aiming device.

Preferably, in addition to the form-fit connection between the adaptor device and the body implant the locking element causes also a force-fit connection between the body implant and the adaptor device by pressing these parts against each other. Thereby, the mechanical connection between the implant and the adaptor device may be improved with respect to the spatially precision and/or with respect to the mechanical stability under load.

The locking element may be an edge or any other kind of projection, which is suitable to be engaged with a locking device.

According to a further embodiment of the present invention the locking element is an internal screw thread. This has the advantage that the locking mechanism of the adaptor device might be realized by means of a simple screw. Thereby, a stable mechanical connection between the adaptor device and the implant may be achieved by pressing these parts mutually against each other. Thereby, both a form-fit and a force-fit connection between the adaptor device and the implant may be realized.

According to a further aspect of the invention there is provided an adaptor device for detachably establishing a spatial orientation between a body implant and an aiming device, in particular between a bone stabilization implant and an aiming device for anchoring the implant to an internal part of a body. The adaptor device comprises (a) an end portion, which is adapted to be detachably fastened to a contour portion of the body implant by means of a mechanic form-fit connection, and (b) a coupling portion, which is adapted to be mechanically connected to the aiming device.

Also this aspect of the present invention is based on the idea that a precise form-fit connection between the body implant and the adaptor device can be realized if the adaptor device and the corresponding contour portion formed at the body implant spatially interact with each other in such a manner that these parts mutually intervene and/or engage with each other. Thereby, a precise relative spatial orientation of the body implant and the adaptor device can be maintained.

The adaptor device comprises at least at one side a three-dimensional surface profile, which is formed in such a manner that it corresponds in a complementary manner to the contour portion of the body implant. Thereby, the adaptor device and the body implant mutually engage with each other.

The adaptor device may be formed integrally from a single material or a material composition. However, the adaptor device may alternatively be assembled from different elements.

According to this second aspect of the present invention the end portion is assigned to a dowel pin, which is designed in such a manner that the dowel pin can be detachably fastened to a central opening of the contour portion. Thereby, the central opening might interact with the adaptor device like a mortise and a tenon such that a relative position between the body implant and the adaptor device is defined in a spatially precise manner. The central opening preferably has a circular respectively a cylindrical shape such that the opening can easily be formed within the implant.

Preferably, the dowel pin may be fastened to the body implant by inserting one end of the dowel pin into the central opening.

According to a further embodiment of the present invention the dowel pin comprises a locking mechanism which is designed in such a manner that the adaptor device is lockable to the body implant by means of a locking element assigned to the body implant.

The provision of a locking mechanism has the advantage that an unintentional opening of the mechanical connection between the adaptor device and the body implant may be prevented effectively. Of course, the adaptor device may be removed from the implant by an appropriate procedure after a surgery procedure using the aiming device has been successfully completed.

In order to provide for a stable connection between the body implant, in particular the contour portion, and the adaptor element, in particular the dowel pin, the contour portion may comprise an edge or any other kind of projection, which is suitable to be engaged with the locking mechanism.

According to a further embodiment of the present invention the locking mechanism comprises an external screw thread. Preferably, the dowel pin might comprise an external screw thread at least at one end portion of the dowel pin. This has the advantage that a mechanically stable connection between the body implant and the adaptor device might be realized by simply screwing the dowel pin into a threaded central opening of the contour portion. Thereby, a stable mechanical connection between the adaptor device and the implant may be achieved by means of a very simple and effective fastening procedure.

According to this second aspect of the present invention the coupling portion is assigned to a handling device comprising a through hole such that the handling device is slideable over the dowel pin. This may provide the advantage that the adaptor device can be easily attached to the implant whereby an appropriate relative orientation between the adaptor device and the implant is automatically ensured.

According to a further embodiment of the present invention the handling device comprises a handle. The handle may protrude laterally from the central part of the handling device which central part is equipped with the through hole. This may provide the advantage that during an operation a manual handling of both the adaptor device and the body implant detachably fastened to the adaptor device is facilitated. Preferably, the handle comprises an ergonomic handhold.

According to this second aspect of the present invention the handling device comprises three protrusions. Each protrusion is formed in such a way that the adaptor device is capable of engaging into a groove of the contour portion of the body implant. Each protrusion is formed

in such a manner that it might interact with a corresponding groove formed within the body implant. This interaction may be similar to the well-known mechanical interaction between tongue and groove. Thereby, also a rotational movement of the adaptor device with respect to the body implant may effectively be prevented.

According to this second aspect of the present invention the handling device comprises two further protrusions. This may provide the advantage that the relative spatial position between the adaptor device and the body implant can be established even more precisely and in a more reliably way.

In this respect it is clear that the contour portion of the body implant has to be formed in a complementary shape with respect to the relevant surface of the handling device such that the adaptor device form-fits to the central opening and to the three grooves.

According to this second aspect of the present invention the protrusions are arranged in an asymmetric manner with respect to the central opening. This may provide the advantage that adaptor device and the body implant can be mechanically connected to each other only in a predefined relative angular orientation. Therefore, an accidentally wrong assembling may be prevented.

According to a further embodiment of the present invention the adaptor device further comprises a clamping element, which is adapted for being mounted onto the dowel pin in order to maintain the handling device in a position wherein the handling device abuts against the body implant.

In case the handling device comprises one ore more protrusions, which engage into corresponding grooves formed within the body implant, the clamping element is capable of reliably securing the handling device and the implant in a spatially stable relative position with respect to each other.

According to a further embodiment of the present invention the clamping element is a nut. Preferably, the clamping element is a cap nut such that the upper portion of the dowel pin is protected and the adaptor device exhibits no sharp edges or corners.

The nut may be provided with an internal screw thread. Provided that the dowel pin is equipped with a corresponding external screw thread this allows for both an easy and a secure fastening of the handling device to the body implant.

According to a further embodiment of the present invention the handling device is designed in such a manner that the aiming device is detachably attachable to the handling device. This may provide the advantage that a predefined relative spatial position between the aiming device and the body implant may be established precisely.

This is in particular helpful if the implant is a plate, which is used for bone stabilization. Such a plate may be used in order to fix a broken bone in its anatomical correct position. Thereby, the plate is attached to different parts of the bone by means of screws, pins, loops, et cetera. In particular when screws are used for attaching the plate, an aiming device may be very helpful, which aiming device comprises a drilling jig. Thereby, an operator and in particular a surgeon is capable of precisely drilling holes into the bone, whereby each hole is formed exactly at the proper position with respect to a corresponding through hole formed in the plate.

According to a further aspect of the invention there is provided a medical system for detachably establishing a spatial orientation between a body implant and an aiming device, in particular between a bone stabilization implant and an aiming device for anchoring the implant to an internal part of a body. The medical system comprises a body implant according to an aspect or an embodiment described above. The medical system further comprises an adaptor device according to an aspect or an embodiment described above.

According to a further aspect of the invention there is provided a method for detachably establishing a spatial orientation between a body implant and an aiming device, in particular between a bone stabilization implant and an aiming device for anchoring the implant to an internal part of a body. The described method comprises (a) fastening a dowel element to a contour portion of the body implant, (b) fixing a handling device to the dowel element such that the handling device abuts against the body implant in a form-fit manner, (c) mounting a clamping element onto the dowel element in order to secure the relative spatial position between the handling device and the body implant, and (d) attaching the aiming device to the handling device.

This aspect of the invention is based on the idea that a mechanically stable spatial relative position between the body implant and the aiming device can be established by means of a multi-step process. First, there is established a precise spatial position between the handling device and the body implant by fixing the handling device to the dowel element fastened to the contour portion of the body implant. Second, there is established a precise spatial position between the handling device and the aiming device by attaching the aiming device to the handling device in an appropriate spatial orientation.

According to an embodiment of the invention the step of fastening a dowel element to a contour portion of the body implant comprises inserting a dowel pin into a central opening of the contour portion. Using a dowel pin as the dowel element may provide the advantage that the dowel pin can be used both (a) as a centering element in order to provide for an easy assembling of the adaptor device and (b) as a mechanical fastener in order to provide for a stable and reliable mechanical connection between the handling device and the body implant.

According to a further embodiment of the present invention the step of inserting a dowel pin into a central opening of the contour portion comprises screwing the dowel pin into an internal screw thread, which is provided in the central opening. As has already been mentioned above, this has the advantage that the locking mechanism of the adaptor device might be realized by means of a simple screw thread. Thereby, a stable mechanical connection between the adaptor device and the body implant may be achieved.

According to a further embodiment of the invention the step of fixing a handling device to the dowel element comprises (a) using a handling device comprising a through hole and (b) sliding the handling device over the dowel pin. This may provide the advantage that an easy and mechanical stable connection between the handling device and the body implant can be established.

According to a further embodiment of the present invention the step of sliding the handling device over the dowel pin comprises rotating the handling device around the dowel pin such that a protrusion, which is provided at the handling device, engages into a groove which is provided at the contour portion.

Preferably, the rotating movement is a bidirectional swivel movement, which is accomplished in the course of the sliding movement in particular when the sliding movement has already come to an end. The described rotational movement may provide the advantage that an engagement between complementary three-dimensional surface portions of the body implant and the handling device, respectively, may be achieved by means of a simple manual movement of the handling device with respect to the body implant.

It has to be noted that embodiments of the invention have been described with reference to different subject matters. In particular, some embodiments have been described with reference to apparatus type claims whereas other embodiments have been described with reference to method type claims. However, a person skilled in the art will gather from the above and the following description that, unless other notified, in addition to any combination of features belonging to one subject matter also any combination between features relating to different subject matters, in particular between features of the apparatus type claims and features of the method type claims is disclosed with this application.

The aspects defined above and further aspects of the present invention are apparent from the examples of embodiment to be described hereinafter and are explained with reference to the examples of embodiment. The invention will be described in more detail hereinafter with reference to examples of embodiment but to which the invention is not limited.

### BRIEF DESCRIPTION OF THE DRAWINGS

Fig. 1a shows a bone stabilizing plate representing a body implant according to an embodiment of the invention.
Fig. 1b shows the upper part of the plate depicted in Fig. 1a.
Fig. 1c shows a cross sectional view of a central opening formed within the plate depicted in Fig. 1a.
Fig. 2 shows an exploded view of a medical system comprising the plate depicted in Fig. 1a and an adaptor device for holding an aiming device in a spatially precise orientation with respect to the plate.
Figs. 3a to 3g illustrate a procedure for assembling the medical system depicted in Fig. 2.

### DETAILED DESCRIPTION

The illustration in the drawing is schematically. It is noted that in different figures, similar or identical elements are provided with the same reference signs or with reference signs, which are different from the corresponding reference signs only within the first digit.

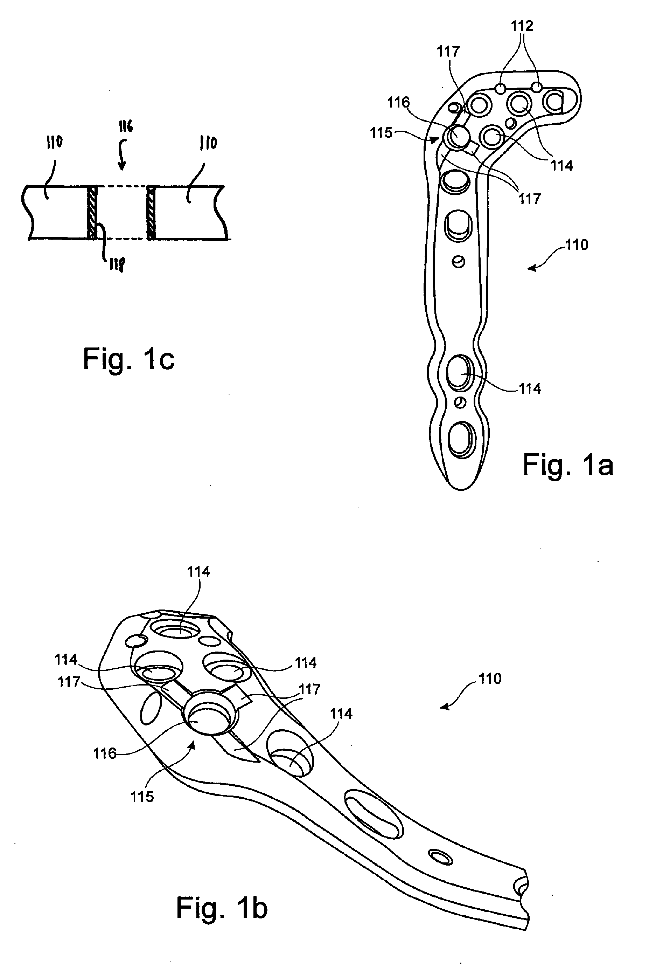

Fig. 1a and Fig. 1b show a bone stabilizing plate 110 representing a body implant 110 according to an embodiment of the invention. Fig. 1b depicts a slightly enlarged view of the upper part of the plate 110.

The bone stabilizing plate 110 comprises a plurality of through holes 114. The through holes 114 may be used for inserting screws (not depicted) for fixing the plate 110 to a bone or to a bone fragment such that all parts of the bone are attached to the bone stabilizing plate 110.

The plate 110 further comprises a contour portion 115, which is a three-dimensional profiled surface portion of an upper surface of the plate 110. The contour portion 115 comprises a central opening 116 and three grooves 117, which stick out radially from the central opening 116.

As can be seen from Fig. 1c which depicts a cross sectional view of the central opening 116 formed within the plate 110, the internal side walls of the central opening 116 are provided with an internal screw thread 118.

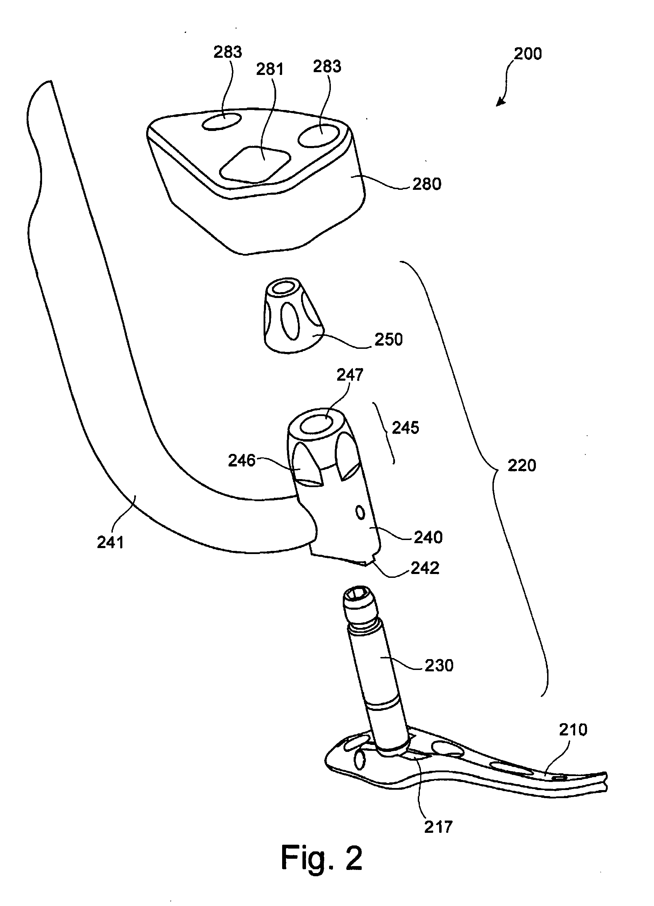

Fig. 2 shows an exploded view of a medical system 200 comprising the plate 210 depicted in Fig. 1a and an adaptor device 220 for holding an aiming device 280 in a spatially precise orientation with respect to the plate 210. The adaptor device 220 comprises several parts, a dowel pin 230, a handling device 240 and a clamping element 250.

The dowel pin 230 has the shape of an elongated shaft. A lower end of the shaft 230 is provided with an external screw thread such the shaft 230 can be screwed into the central opening 116 of the plate 210, which central opening is provided with a internal screw thread 118.

The handling device 240 comprises a through hole 247 such that the dowel pin 230 might be inserted into the handling device 240. In other words, the handling device 240 might be pinned up onto the dowel pin 230.

The handling device 240 further comprises a handle 241, which preferably is formed in such a manner that the handle 241 represents an ergonomic handhold. This provides the advantage that during an operation a manual handling of the whole medical system 200 is facilitated.

In order to provide for a stable mechanical connection between the handling device 240 and the plate 210 a lower surface of the handling device 240 is formed in such a manner that a three-dimensional profiled surface is provided, which corresponds in a complementary way to the contour portion 115 of the plate 210. According to the embodiment described here the three-dimensional profiled contour comprises three protrusions 242, which respectively engage within a corresponding groove 217 formed within the body implant 210. An engagement of the protrusions 242 with the corresponding grooves 217 has the effect, that a rotational movement of the handling device 240 with respect to the dowel pin 230 is effectively prevented.

According to the embodiment described here, the grooves 117, which might also be denominated as positioning slots, have a maximum depth of approximately 0.7 mm. However, also other dimensions for the grooves 117 may be applicable.

The protrusions 242 and consequently also the grooves 217 are arranged in an asymmetric manner around the central opening respectively the through hole 247. This has the effect that the handling device 240 and the body implant 210 can be mechanically connected to each other only in a predefined relative angular orientation. Therefore, an accidentally wrong assembling of these parts is automatically prevented.

It has to be mentioned that the protrusions 242 respectively the grooves 217 protrude from the through hole 247 respectively the central opening 116 in such a manner that when a typical bending load is exerted between the plate 210 and the handling device 240 the dowel pin 230 is subjected to a tensile stress only. Thereby, the stability of the whole adaptor device is improved.

The handling device 240 furthermore comprises a coupling portion 245, which is formed at an upper section of the handling device 240. The coupling portion 245 is represented by a special shape of the outer surface 246 of the handling device 240. This outer surface 246 is formed in such a manner that it complementary fits to an inner surface (not depicted) of an opening 281 formed within an aiming device 280. Thereby, the aiming device may be detachably fastened to the handling device 240 by pushing the aiming device 280 into the proper position on the coupling portion 245 of the handling device 240. In order to provide for a secure but detachable connection the aiming device 280 is connected to the handling device 240 by means of a so-called quick coupling device which are well known to men skilled in the art.

In order to prevent that the aiming device 280 is assembled to the coupling portion 245 in the wrong position the outer surfaces 246 of the handling device 240 are distributed in a rotational asymmetric manner.

The clamping element 250 is adapted for being mounted onto the dowel pin 230 in order to maintain the handling device 240 in a position wherein the handling device 240 abuts against the body implant 210. Since the handling device 240 comprises three protrusions 242, which engage into corresponding grooves 217, the clamping element 250 is capable of reliably securing the handling device 240 and the plate 210 in a spatially stable relative position with respect to each other.

As can be seen from Fig. 2, the clamping element is a cap nut such that the upper portion of the dowel pin 230 may be covered. This has the advantage that in an assembled state no edges of the dowel pin 230 project or protrude above the upper surface of the aiming device 280.

Preferably, the dowel pin 230 is provided with a spring ring (not depicted). The spring ring is adapted to engage into a corresponding snap ring groove (not depicted), which is provided at the inner side wall of the through hole 247 formed within the handling device 240. This has the advantage that during an assembling procedure of the adaptor device 220 the handling device 240 is temporarily fixed to the dowel pin 230 by means of a snapping mechanism already before the cape nut 250 is completely screwed onto the external screw thread of the dowel pin 230. Thereby, the assembling procedure of the adaptor device 220 may be facilitated significantly.

The aiming device 280 represents a drilling jig comprising an opening 281 for being connected with the coupling portion of the handling device 240. The aiming device 280 further comprises several through holes 283 for a precise and easy pre drilling of holes into a bone fragment, which is located directly beneath the bone stabilizing plate 210. Thereby, the through holes 283 represent a drilling jig allowing for a minimal invasive and percutaneous surgery technique. When the aiming device 280 is properly mounted to the handling device 240 the holes 283 of the aiming device 280 are aligned with the holes 214 in the plate 210.

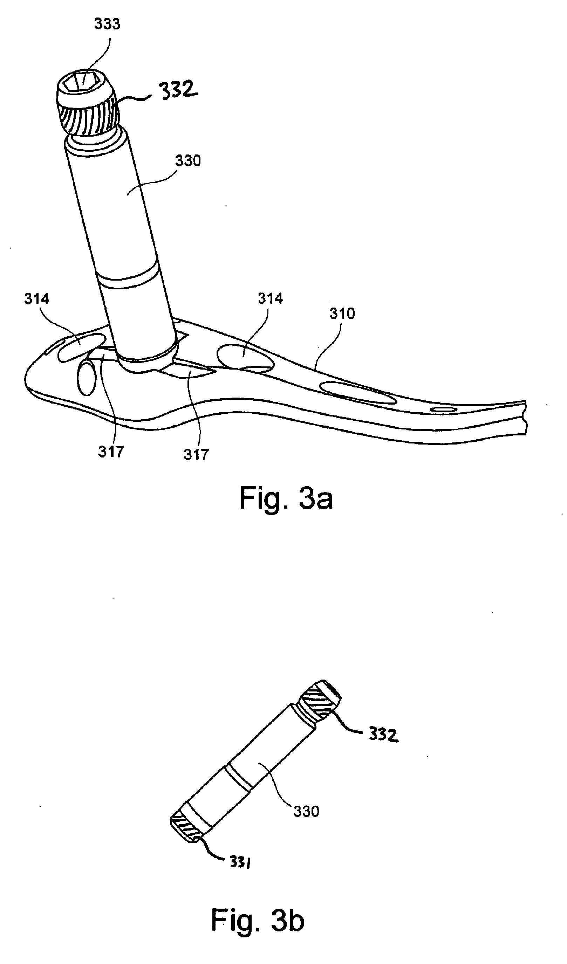

Figs. 3a to 3g illustrate a procedure for assembling the medical system 200 depicted in Fig. 2.

As can be seen from Fig. 3a the assembling starts with inserting the dowel pin 330 into the central opening 116 (see Fig. 1) of the bone stabilizing plate 310, which comprises several through holes 314. The relevance of the through holes 314 has already been explained above.

In order to provide for both a spatially stable and a mechanically reliable connection between the plate 310 and the dowel pin 330, the outer surface at the lower portion of the dowel pin 330 is provided with an external screw thread 331. This thread corresponds to an internal screw thread 118 provided at the inner side wall of the central opening 116 (see Fig. 1). Therefore, the dowel pin 330 is fastened to the plate 310 by screwing the dowel pin 330 into the central opening 116. In order to facilitate the screwing the upper portion of the dowel pin 330 is provided with a drive socket 333.

The dowel pin 330 is depicted individually in Fig. 3b. As has already been described above the dowel pin 330 has the shape of an elongated shaft. A lower end of the shaft 330 is provided with an external screw thread 331 such the shaft 330 can be screwed into the central opening 116 of the plate 310, which central opening is provided with a internal screw thread 118. Also an upper end of the shaft 330 is provided with an external screw thread 332 such the cap nut 250 depicted in Fig. 2 can be screwed onto the dowel pin 330.

According to the embodiment described here the dowel pin 330 is a hollow shaft having an outer diameter of approximately 6.0 mm and an inner diameter of approximately 2.6 mm. However, also other dimensions for the dowel pin 330 may be applicable.

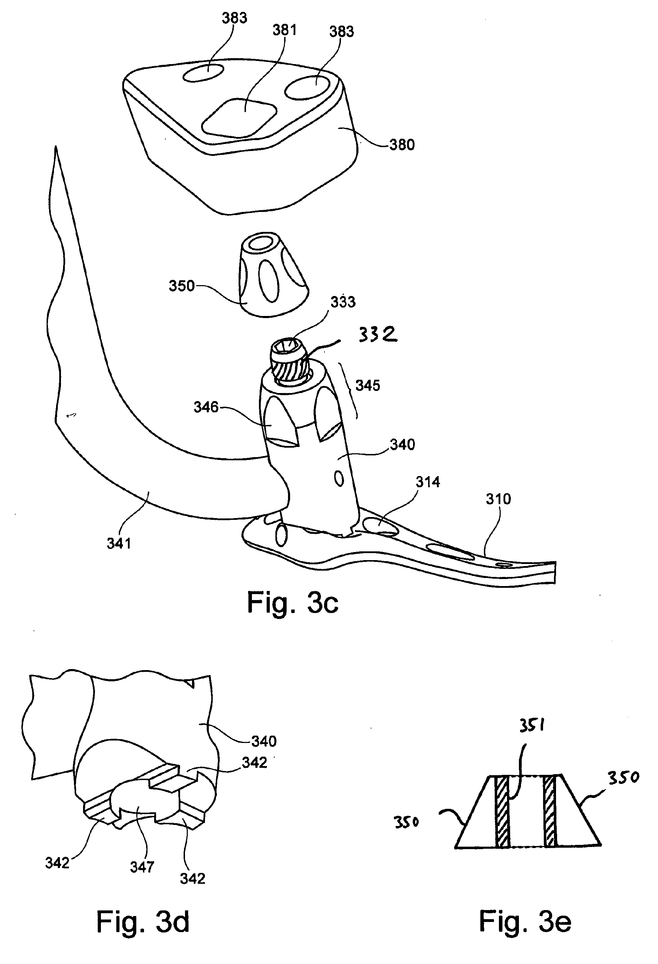

Fig. 3c shows the medical system in an advanced assembled state. A handling device 340 is pinned on the dowel pin 330. As can be seen from the small inset in Fig. 3b, which depicts the lower surface of the handling device 340, the handling device 340 comprises an opening 347 for receiving the dowel pin 330 and three protrusions 342 for being engaged into the grooves 317 of the plate 310.

When being assembled, the handling device 340 abuts against the contoured portion of the plate 310 such that the protrusions 342 engage into the grooves 317. In order to quickly find the correct relative angular position between the protrusions 342 of the handling device 340 and the grooves 317 of the plate 310, the handling device 340 is rotated around the dowel pin when the handling device 340 is pushed over the dowel pin 330. The handling of the handling device 340 is facilitated by the handle 341, which is formed at the handling device 340.

Fig. 3d shows an enlarged view of the lower surface of the handling device 340. This surface is provided with three protrusions 342, which project from the lower surface. In order to prevent that the handling device 340 is attached to the plate 310 in a wrong angular position the protrusions 342 are arranged in an asymmetric T-form.

Fig. 3e shows a cross sectional view of the cap nut 350. In order to allow for a reliable and stable connection between the plate 310 and the handling device 340 the cap nut 350 is provided with an internal screw thread 351. Provided that the dowel pin 330 is equipped with a corresponding external screw thread 332 this allows for both an easy and a secure fastening of the handling device 340 to the body implant 310.

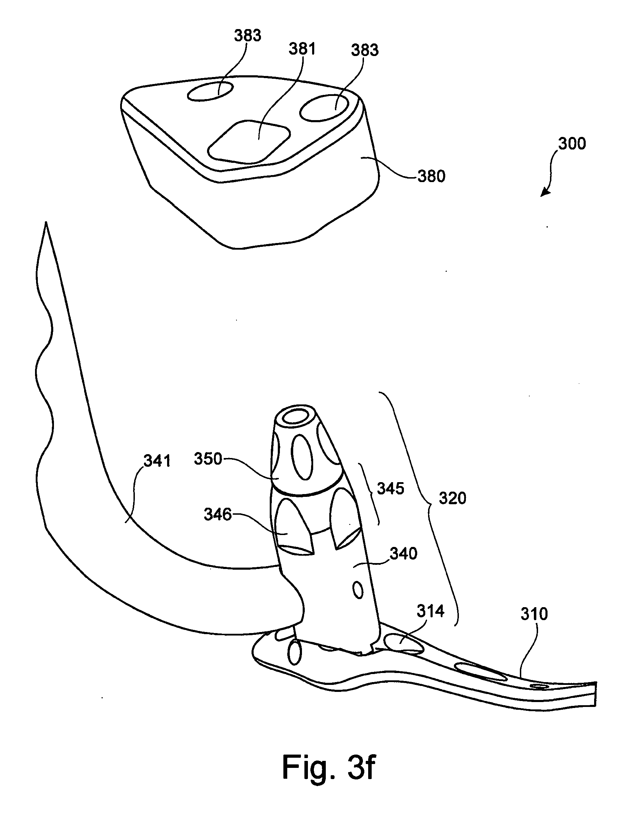

Fig. 3f shows the medical system 300 in a further advanced assembled state. A clamping element 350 is fastened to the upper portion of the dowel pin 330. The clamping element is a nut 350 having an internal screw thread 351 that fits to a corresponding external screw thread 332 provided at the outer surface of the upper section of the dowel pin 330. Therefore, by screwing the nut 350 onto the dowel pin 330 the handling device 340 is pressed against the plate 310. Thereby, also a force-fit connection between the handling device 340 and the plate 310 is provided such the relative spatial position between these two parts 340 and 310 being precisely defined by the protrusions 342 and the corresponding grooves 317 can be maintained even under a mechanical load.

In this respect it is pointed out that the protrusions 342 and the corresponding grooves 317 cause a form-fit connection between the handling device 340 and the plate 310 whereas the nut 350 being threaded screwed onto the dowel pin 330 and the dowel pin 330 being screwed into the plate 310 causes a form-fit connection between the handling device 340 and the plate 310.

In order to provide a basis for an aiming device 380, the handling device 340 comprises a coupling portion 345. An outer surface 346 is formed in such a manner that it complementary fits to an inner surface (not depicted) of an opening 381 formed within the aiming device 380. Thereby, the aiming device may be detachably fastened to the handling device 340 by pushing the aiming device 380 into the proper position on the coupling portion 345.

In the assembled state the dowel pin 330, the handling device 340 and the clamping element 350 represent an adaptor device 320, which allows for a precise and stable positioning of the aiming device 380 with respect to the plate 310.

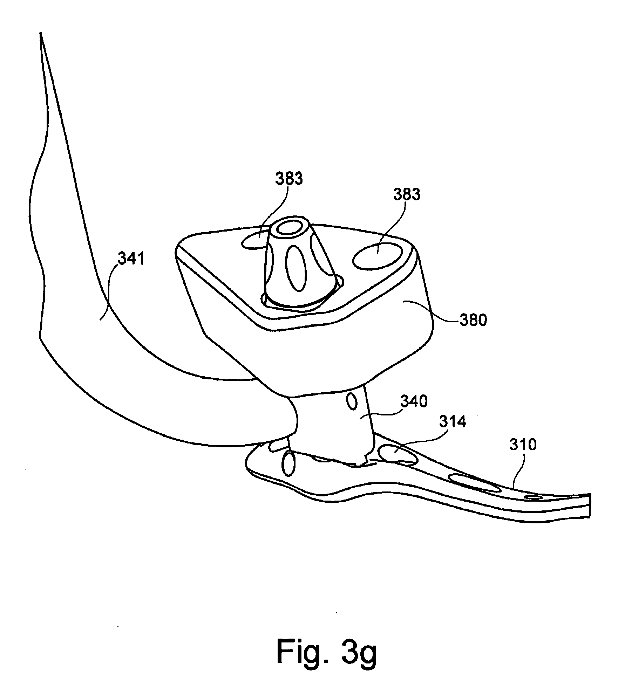

Fig. 3g shows the medical system 300 in the final assembled state. The aiming device 380 is pushed into the proper position onto the coupling portion 345. In order to provide for a precise and easy pre drilling of holes into a bone fragment, which is located directly beneath the bone stabilizing plate 310, the aiming device 380 is provided with an opening 381 and several through holes 383. The spatial arrangement of the through holes 383 represents a drilling jig. Therefore, the aiming device helps a surgeon for pre-drilling holes into a broken bone exactly at positions, which correspond to the through holes 314 of the plate 310.

It should be noted that the term "comprising" does not exclude other elements or steps and the "a" or "an" does not exclude a plurality. Also elements described in association with different embodiments may be combined. It should also be noted that reference signs in the claims should not be construed as limiting the scope of the claims.

In order to recapitulate the above described embodiments of the present invention one can state:
It is described a medical system 200, system parts 210, 220 and a method for detachably establishing a spatial orientation between a body implant 210 and an aiming device 280. Thereby, a contoured portion 115 of a body implant 110, 210 is designed in such a manner that an adaptor device 220 can be detachably fastened to the body implant 110, 210 by means of a mechanic form-fit connection. The adaptor device 220 provides a basis for an aiming device 280 such that the aiming device 280 may be detachably positioned in a stable spatial position with respect to the body implant 210. Preferably, the adaptor device 220 comprises different elements like a dowel pin 230, a handling device 240 and a clamping element 250, which elements allow for a quick and easy assembling and disassembling of the adaptor device 220.

### List of reference signs:

- 110: bone stabilizing plate / body implant
- 114: through holes
- 115: contour portion
- 116: central opening
- 117: grooves
- 118: locking element / internal screw thread

- 200: medical system
- 210: bone stabilizing plate / body implant
- 217: grooves
- 220: adaptor device
- 230: dowel pin / elongated shaft
- 231: locking mechanism / external screw thread
- 240: handling device
- 241: handle
- 242: end portion / protrusion
- 245: coupling portion
- 246: outer surface of the handling device 240
- 247: through hole
- 250: clamping element / nut
- 280: aiming device
- 281: opening
- 283: though holes / drilling jig
- 300: medical system
- 310: bone stabilizing plate / body implant
- 314: through holes
- 317: grooves
- 320: adaptor device
- 330: dowel pin
- 331: external screw thread
- 332: external screw thread
- 333: drive socket
- 340: handling device
- 341: handle
- 342: protrusion
- 345: coupling portion
- 346: outer surface of the handling device 340
- 347: through hole
- 350: clamping element / nut
- 351: internal screw thread
- 380: aiming device
- 381: opening
- 383: though holes / drilling jig

## Claims

1. A body implant, in particular a bone stabilizing implant (110, 210), comprising: through holes (114) for anchoring the implant (110, 210) to an internal part of a body by means of fastening elements and a contour portion (115) which is designed in such a manner that an adaptor device (220) for establishing a spatial orientation between the body implant (110, 210) and an aiming device (280) can be detachably fastened to the body implant (110, 210) by means of a mechanic form-fit connection, wherein the contour portion (115) comprises a central opening (116), **characterized in that** the contour portion (115) further comprises three grooves (117, 217), wherein the central opening (116) is a through hole, and wherein the three grooves (117) are arranged in an asymmetric manner with respect to the central opening (116).

2. The body implant according to claim 1, wherein the contour portion (115) comprises a locking element (118) which is designed in such a manner that the adaptor device (220) is lockable to the body implant (110, 210) by means of a corresponding locking mechanism (331) at the adaptor device (220).

3. The body implant according to claim 2, wherein the locking element (118) is an internal screw thread.

4. An adaptor device for detachably establishing a spatial orientation between a body implant (110, 210) and an aiming device (280), in particular between a bone stabilization implant (110, 210) and an aiming device (280) for anchoring the implant (110, 210) to an internal part of a body, the adaptor device (220) comprising a dowel pin (230) and a handling device (240),
wherein the dowel pin (230) is designed in such a manner that the dowel pin (230) can be detachably fastened to a central opening (116) of the contour portion (115) of the body implant,
wherein the handling device (240) includes an end portion (242), a coupling portion (245) and a through hole (247) such that the handling device (240) is slideable over the dowel pin (230),
wherein the end portion (242) is adapted to be detachably fastened to a contour portion (115) of the body implant (110, 210) by means of a mechanic form-fit connection, wherein three protrusions (242) are arranged at the end portion (242) in an asymmetric manner with respect to the central opening (116), and are formed in such a way that the end portion (242) is capable of engaging into corresponding grooves (117, 217) of the contour portion (115) of the body implant (110, 210),
and
wherein the coupling portion (245) is adapted to be mechanically connected to the aiming device (280).

5. The adaptor device according to claim 4, wherein
the dowel pin (230) comprises a locking mechanism (331) which is designed in such a manner that the adaptor device (220) is lockable to the body implant (210) by means of a corresponding locking element (118) at the body implant (210).

6. The adaptor device according to claim 5, wherein
the locking mechanism (331) comprises an external screw thread.

7. The adaptor device according to claim 4, wherein
the handling device (240) comprises a handle (241).

8. The adaptor device according to any one of the claims 4 to 7, further comprising a clamping element (250), which is adapted for being mounted onto the dowel pin (230) in order to maintain the handling device (240) in a position wherein the handling device (240) abuts against the body implant (210).

9. The adaptor device according to claim 8, wherein the clamping element is a nut (250).

10. The adaptor device according to any one of the claims 4 to 9, wherein the handling device (240) is designed in such a manner that the aiming device (280) is detachably attachable to the handling device (240).

11. A medical system for detachably establishing a spatial orientation between a body implant (110, 210) and an aiming device (280), in particular between a bone stabilization implant (110, 210) and an aiming device (280) for anchoring the implant (110, 210) to an internal part of a body, the system comprising:
a body implant (110, 210) according to any one of the claims 1 to 3, and
an adapter device (220) according to any one of the claims 4 to 10.

12. A method for detachably establishing a spatial orientation between a body implant (110, 210) and an aiming device (280), in particular between a bone stabilization implant (110, 210) and an aiming device (280), the method comprising: fastening a dowel pin (230) to a contour portion (115) of the body implant (110, 210), fixing a handling device (240) to the dowel pin (230) such that the handling device (240) abuts against the body implant (110, 210) in a form-fit manner, mounting a clamping element (250) onto the dowel pin (230) in order to secure the relative spatial position between the handling device (240) and the body implant (210), and attaching the aiming device (280) to the handling device (240),
wherein the contour portion (115) comprises a central opening (116) and three grooves (117, 217), and wherein the three grooves (117) are arranged in an asymmetric manner with respect to the central opening (116).

13. The method according to claim 12, wherein fastening a dowel element (230) to a contour portion (115) of the body implant (110, 210) comprises inserting a dowel pin (230) into a central opening (116) of the contour portion (115).

14. The method according to claim 13, wherein
inserting a dowel pin (230) into a central opening (116) of the contour portion (115) comprises
screwing the dowel pin (230) into an internal screw thread (118) which is provided in the central opening (116).

15. The method according to claim 14, wherein
fixing a handling device (240) to the dowel element (230) comprises
using a handling device (240) comprising a through hole (247) and
sliding the handling device (240) over the dowel pin (230).

16. The method according to claim 15, wherein
sliding the handling device (240) over the dowel pin (230) comprises
rotating the handling device (240) around the dowel pin (230) such that a protrusion (242), which is provided at the handling device (240), engages into a groove (217), which is provided at the contour portion (115).

## Patentansprüche

1. Körperimplantat, insbesondere knochenstabilisierendes Implantat (110, 210), umfassend: durchgehende Öffnungen (114) zum Verankern des Implantats (110, 210) an einem internen Teil eines Körpers unter Einsatz von Befestigungselementen und einem Konturabschnitt (115), der derart gestaltet ist, dass eine Adaptervorrichtung (220) zum Festlegen einer räumlichen Ausrichtung zwischen dem Körperimplantat (110, 210) und der Zielvorrichtung (280) unter Einsatz von mechanischer, formschlüssiger Verbindung lösbar an dem Körperimplantat (110, 210) befestigt werden kann, wobei der Konturabschnitt (115) eine mittige Öffnung (116) umfasst, **dadurch gekennzeichnet, dass** der Konturabschnitt (115) ferner drei Einkerbungen (117, 217) umfasst, wobei die mittige Öffnung (116) eine durchgehende Öffnung ist und wobei die drei Einkerbungen (117) bezüglich der mittigen Öffnung (116) asymmetrisch angeordnet sind.

2. Körperimplantat nach Anspruch 1, wobei der Konturabschnitt (115) ein Verriegelungselement (118) umfasst, welches derart gestaltet ist, dass die Adaptervorrichtung (220) unter Einsatz eines entsprechenden Verriegelungsmechanismus (331) an der Adaptervorrichtung (220) verriegelbar am Körperimplantat (110, 210) angebracht werden kann.

3. Körperimplantat nach Anspruch 2, wobei das Verriegelungselement (118) ein Innengewinde ist.

4. Adaptervorrichtung zum lösbaren Festlegen einer räumlichen Ausrichtung zwischen einem Körperimplantat (110, 210) und einer Zielvorrichtung (280), insbesondere zwischen einem knochenstabilisierenden Implantat (110, 210) und einer Zielvorrichtung (280) zum Verankern des Implantats (110, 210) an einem internen Teil eines Körpers, wobei die Adaptervorrichtung (220) einen Spannstift (230) und eine Handhabungsvorrichtung umfasst (240),
wobei der Spannstift (230) derart gestaltet ist, dass der Spannstift (230) lösbar an einer mittigen Öffnung (116) des Konturabschnitts (115) des Körperimplantats befestigt werden kann,
wobei die Handhabungsvorrichtung (240) einen Endabschnitt (242), einen Kopplungsabschnitt (245) und eine durchgehende Öffnung (247) hat, sodass die Handhabungsvorrichtung (240) über den Spannstift (230) verschiebbar ist,
wobei der Endabschnitt (242) angepasst ist, um unter Einsatz einer mechanischen formschlüssigen Verbindung lösbar an einem Konturabschnitt (115) eines Körperimplantats (110, 210) befestigt zu werden, wobei drei Ausbuchtungen (242) bezüglich der mittigen Öffnung (116) so asymmetrisch am Endabschnitt (242) angeordnet sind, dass der Endabschnitt (242) in die entsprechenden Einkerbungen (117, 217) des Konturabschnitts (115) des Körperimplantats (110, 210) eingreifen kann, und
wobei der Kopplungsabschnitt (245) angepasst ist, um mechanisch mit der Zielvorrichtung verbunden zu werden (280).

5. Adaptervorrichtung nach Anspruch 4, wobei
der Spannstift (230) einen Verriegelungsmechanismus (331) umfasst, der derart gestaltet ist, dass die Adaptervorrichtung (220) unter Einsatz eines entsprechenden Verriegelungselements (118) am Körperimplantat (210) verriegelbar am Körperimplantat (210) befestigt werden kann.

6. Adaptervorrichtung nach Anspruch 5, wobei
der Verriegelungsmechanismus (331) ein Außengewinde umfasst.

7. Adaptervorrichtung nach Anspruch 4, wobei
die Handhabungsvorrichtung (240) einen Griff (241) umfasst.

8. Adaptervorrichtung nach einem der Ansprüche 4 bis 7, ferner umfassend ein Klemmelement (250), welches angepasst ist, um auf dem Spannstift (230) befestigt zu werden, um die Handhabungsvorrichtung (240) in einer Position zu halten, in der die Handhabungsvorrichtung (240) am Körperimplantat (210) anliegt.

9. Adaptervorrichtung nach Anspruch 8, wobei das Klemmelement eine Mutter (250) ist.

10. Adaptervorrichtung nach einem der Ansprüche 4 bis 9, wobei die Handhabungsvorrichtung (240) derart gestaltet ist, dass die Zielvorrichtung (280) lösbar an der Handhabungsvorrichtung (240) befestigt ist.

11. Medizinisches System zum lösbaren Festlegen einer räumlichen Ausrichtung zwischen einem Körperimplantat (110, 210) und einer Zielvorrichtung (280), insbesondere zwischen einem knochenstabilisierenden Implantat (110, 210) und einer Zielvorrichtung (280) zum Verankern des Implantats (110, 210) an einem internen Teil eines Körpers, wobei das System umfasst:
ein Körperimplantat (110, 210) nach einem der Ansprüche 1 bis 3, und
einer Adaptervorrichtung (220) nach einem der Ansprüche 4 bis 10.

12. Verfahren zum lösbaren Festlegen einer räumlichen Ausrichtung zwischen einem Körperimplantat (110, 210) und einer Zielvorrichtung (280), insbesondere zwischen einem knochenstabilisierenden Implantat (110, 210) und einer Zielvorrichtung (280), wobei das Verfahren Folgendes umfasst: Befestigen eines Spannstifts (230) an einem Konturabschnitt (115) des Körperimplantats (110, 210); Befestigen einer Handhabungsvorrichtung (240) am Spannstift (230), sodass die Handhabungsvorrichtung (240) formschließend am Körperimplantat (110, 210) anliegt; Befestigen eines Klemmelements (250) am Spannstift (230), um die relative räumliche Position zwischen der Handhabungsvorrichtung (240) und dem Körperimplantat (210) zu sichern; und Anbringen der Zielvorrichtung (280) an der Handhabungsvorrichtung (240),
wobei der Konturabschnitt (115) eine mittige Öffnung (116) und drei Einkerbungen (117, 217) umfasst, und wobei die drei Einkerbungen (117) bezüglich der mittigen Öffnung (116) asymmetrisch angeordnet sind.

13. Verfahren nach Anspruch 12, wobei das Befestigen eines Spannelements (230) an einem Konturabschnitt (115) des Körperimplantats (110, 210) das Einführen eines Spannstifts (230) in eine mittige Öffnung (116) des Konturabschnitts (115) umfasst.

14. Verfahren nach Anspruch 13, wobei
das Einführen eines Spannstifts (230) in eine mittige Öffnung (116) des Konturbereichs (115) Folgendes umfasst:
Eindrehen des Spannstifts (230) in ein Innengewinde (118) welches in der mittigen Öffnung (116) bereitgestellt ist.

15. Verfahren nach Anspruch 14, wobei
das Befestigen einer Handhabungsvorrichtung (240) an dem Spannelement (230) Folgendes umfasst:
Benutzen einer Handhabungsvorrichtung (240), welches eine durchgehende Öffnung (247) umfasst und
Führen der Handhabungsvorrichtung (240) über den Spannstift (230).

16. Verfahren nach Anspruch 15, wobei
das Führen der Handhabungsvorrichtung (240) über den Spannstift (230) Folgendes umfasst:
Drehen der Handhabungsvorrichtung (240) um den Spannstift (230), sodass eine Ausbuchtung (242), die an der Handhabungsvorrichtung (240) bereitgestellt ist, in eine Einkerbung (217), die am Konturabschnitt (115) bereitgestellt ist, eingreift.

## Revendications

1. Implant corporel, en particulier un implant de stabilisation osseuse (110, 210), comportant : des trous traversants (114) pour ancrer l'implant (110, 210) à une partie interne d'un corps au moyen d'éléments de fixation et d'une portion de contour (115) qui est conçue de manière à pouvoir fixer, de manière détachable, un dispositif adaptateur (220) destiné à établir une orientation spatiale entre l'implant corporel (110, 210) et un dispositif de pointage (280), sur l'implant corporel (110, 210) au moyen d'une liaison mécanique par complémentarité de formes, dans lequel la portion de contour (115) comporte une ouverture centrale (116), **caractérisé en ce que** la portion de contour (115) comporte en outre trois rainures (117, 217), dans lequel l'ouverture centrale (116) est un trou traversant, et dans lequel les trois rainures (117) sont agencées de manière asymétrique par rapport à l'ouverture centrale (116).

2. Implant corporel selon la revendication 1, dans lequel la portion de contour (115) comporte un élément de blocage (118) qui est conçu de manière à pouvoir bloquer le dispositif adaptateur (220) sur l'implant corporel (110, 210) au moyen d'un mécanisme de blocage (331) correspondant sur le dispositif adaptateur (220).

3. Implant corporel selon la revendication 2, dans lequel l'élément de blocage (118) est un filetage de vis intérieur.

4. Dispositif adaptateur destiné à établir, de manière détachable, une orientation spatiale entre un implant corporel (110, 210) et un dispositif de pointage (280), en particulier entre un implant de stabilisation osseuse (110, 210) et un dispositif de pointage (280) pour ancrer l'implant (110, 210) à une partie interne d'un corps, le dispositif adaptateur (220) comportant un tenon de guidage (230) et un dispositif de manipulation (240),
dans lequel le tenon de guidage (230) est conçu de manière à pouvoir fixer, de manière détachable, le tenon de guidage (230) à une ouverture centrale (116) de la portion de contour (115) de l'implant corporel,
dans lequel le dispositif de manipulation (240) inclut une portion d'extrémité (242), une portion de couplage (245) et un trou traversant (247) de telle sorte que le dispositif de manipulation (240) peut glisser sur le tenon de guidage (230),
dans lequel la portion d'extrémité (242) est adaptée pour être fixée de manière détachable à une portion de contour (115) de l'implant corporel (110, 210) au moyen d'une liaison mécanique par complémentarité de formes, dans lequel trois saillies (242) sont agencées sur la portion d'extrémité (242) de manière asymétrique par rapport à l'ouverture centrale (116), et sont formées de manière à pouvoir engager la portion d'extrémité (242) dans des rainures (117, 217) correspondantes de la portion de contour (115) de l'implant corporel (110, 210), et
dans lequel la portion de couplage (245) est adaptée pour être mécaniquement reliée au dispositif de pointage (280).

5. Dispositif adaptateur selon la revendication 4, dans lequel le tenon de guidage (230) comporte un mécanisme de blocage (331) qui est conçu de manière à bloquer le dispositif adaptateur (220) sur l'implant corporel (210) au moyen d'un élément de blocage (118) correspondant sur l'implant corporel (210).

6. Dispositif adaptateur selon la revendication 5, dans lequel le mécanisme de blocage (331) comporte un filetage de vis extérieur.

7. Dispositif adaptateur selon la revendication 4, dans lequel le dispositif de manipulation (240) comporte une poignée (241).

8. Dispositif adaptateur selon l'une quelconque des revendications 4 à 7, comportant en outre un élément de serrage (250), qui est adapté pour être monté sur le tenon de guidage (230) afin de maintenir le dispositif de manipulation (240) dans une position dans laquelle le dispositif de manipulation (240) vient en butée contre l'implant corporel (210).

9. Dispositif adaptateur selon la revendication 8, dans lequel l'élément de serrage est un écrou (250).

10. Dispositif adaptateur selon l'une quelconque des revendications 4 à 9, dans lequel le dispositif de manipulation (240) est conçu de manière à pouvoir bloquer le dispositif de pointage (280) sur le dispositif de manipulation (240) de manière détachable.

11. Système médical destiné à établir, de manière détachable, une orientation spatiale entre un implant corporel (110, 210) et un dispositif de pointage (280), en particulier entre un implant de stabilisation osseuse (110, 210) et un dispositif de pointage (280) pour ancrer l'implant (110, 210) à une partie interne d'un corps, le système comportant :
un implant corporel (110, 210) selon l'une quelconque des revendications 1 à 3, et
un dispositif adaptateur (220) selon l'une quelconque des revendications 4 à 10.

12. Procédé pour établir, de manière détachable, une orientation spatiale entre un implant corporel (110, 210) et un dispositif de pointage (280), en particulier entre un implant de stabilisation osseuse (110, 210) et un dispositif de pointage (280), le procédé comportant les étapes consistant à : fixer un tenon de guidage (230) à une portion de contour (115) de l'implant corporel (110, 210), fixer un dispositif de manipulation (240) au tenon de guidage (230) de sorte que le dispositif de manipulation (240) vient en butée contre l'implant corporel (110, 210) par complémentarité de formes, monter un élément de serrage (250) sur le tenon de guidage (230) afin de bloquer la position spatiale relative entre le dispositif de manipulation (240) et l'implant corporel (210), et fixer le dispositif de pointage (280) au dispositif de manipulation (240), **caractérisé en ce que** la portion de contour (115) comporte en outre une ouverture centrale (116) et trois rainures (117, 217), et dans lequel les trois rainures (117) sont agencées de manière asymétrique par rapport à l'ouverture centrale (116).

13. Procédé selon la revendication 12, dans lequel la fixation d'un tenon de guidage (230) à une portion de contour (115) de l'implant corporel (110, 210) comporte l'étape consistant à :
insérer un tenon de guidage (230) dans une ouverture centrale (116) de la portion de contour (115).

14. Procédé selon la revendication 13, dans lequel l'insertion d'un tenon de guidage (230) dans une ouverture centrale (116) de la portion de contour (115) comporte le vissage du tenon de guidage (230) dans un filetage de vis intérieur (118) qui est agencé dans l'ouverture centrale (116).

15. Procédé selon la revendication 14, dans lequel la fixation d'un dispositif de manipulation (240) au tenon de guidage (230) comporte l'utilisation d'un dispositif de manipulation (240) comportant un trou traversant (247) et le glissement du dispositif de manipulation (240) sur le tenon de guidage (230).

16. Procédé selon la revendication 15, dans lequel le glissement du dispositif de manipulation (240) sur le tenon de guidage (230) comporte la rotation du dispositif de manipulation (240) autour du tenon de guidage (230) de telle sorte qu'une saillie (242), qui est agencée sur le dispositif de manipulation (240), s'engage dans une rainure (217), qui est agencée sur la portion de contour (115).
